# EUROPÄISCHE PATENTSCHRIFT

(11) **EP 2 831 122 B1**
(45) Veröffentlichungstag und Bekanntmachung des Hinweises auf die Patenterteilung: **03.02.2016**
(21) Anmeldenummer: 13713428.4
(22) Anmeldetag: 28.03.2013
(51) Int. Cl.: C08B 37/00, C08L 5/16, C08B 37/16

(54) **ANTHOCYANIDIN-KOMPLEX**
ANTHOCYANIDIN COMPLEX
COMPLEXE À BASE D'ANTHOCYANIDINE

(30) Priorität: 30.03.2012 EP 12002350
(43) Veröffentlichungstag der Anmeldung: 04.02.2015
(73) Patentinhaber: SapioTec GmbH, 97082 Würzburg (DE)
(72) Erfinder: ROEWER, Norbert, 97082 Würzburg (DE); BROSCHEIT, Jens, 97209 Würzburg (DE)
(74) Vertreter: Glawe, Delfs, Moll
(86) Internationale Anmeldenummer: PCT/EP2013/056707
(87) Internationale Veröffentlichungsnummer: WO 2013/144297

(56) Entgegenhaltungen:
- WO-A2-2009/134347
- CN-A- 1 672 534
- US-A1- 2011 224 168
- STELLA VALENTINO J ET AL: "Cyclodextrins: Their Future in Drug Formulation and Delivery", PHARMACEUTICAL RESEARCH, KLUWER ACADEMIC PUBLISHERS, NEW YORK, NY, US, Bd. 14, Nr. 5, 1. Mai 1997 (1997-05-01), Seiten 556-567, XP002080397, ISSN: 0724-8741, DOI: 10.1023/A:1012136608249

## Beschreibung

Die Erfindung betrifft einen Komplex aus einem Anthocyanidin und einem Sulfoalkylether-ß-Cyclodextrin.

Anthocyanidine sind zymochrome Farbstoffe, die in den meisten höheren Landpflanzen vorkommen. Anthocyanidine sind zuckerfrei (Aglycone) und eng verwandt mit dem zuckerhaltigen Anthocyanen. Anthocyanidine sind Farbstoffe und besitzen antioxidative Eigenschaften.

CN 1 672 534 A offenbart ein Verfahren zur Gefriertrocknung von Erdbeeren, bei dem eine β-Cyclodextrin-Lösung und eine Cyanidin-Lösung mit frischen Erdbeeren in einen Vakuumbehälter gegeben werden.

Der Erfindung liegt die Aufgabe zu Grunde, Anthocyandine in gut handhabbarer, formulierbarer und lagerstabiler Form zur Verfügung zu stellen.

Gelöst wird diese Aufgabe durch einen Komplex aus einem Anthocyanidin und einem Sulfoalkylether-β-Cyclodextrin.

Zunächst sein einige im Rahmen der Erfindung verwendete Begriffe erläutert.

Anthocyanidine weisen die nachfolgend wiedergegebene Grundstruktur auf.

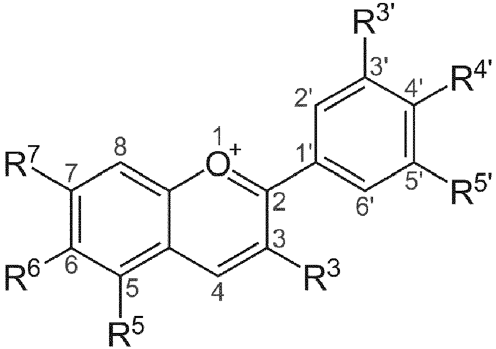

Die Substituenten in dieser Formel sind ausgewählt aus der Gruppe bestehend aus Wasserstoff, Hydroxygruppe und Methoxygruppe.

Cyclodextrine sind zyklische Oligosaccharide aus α-1,4-glykosidisch verknüpften Glukosemolekülen. β-Cyclodextrin besitzt sieben Glukoseeinheiten. Bei einem Sulfoalkyether-ß-Cyclodextrin sind Hydroxygruppen der Glukoseeinheit in einem Sulfoalkylalkohol verethert. Erfindungsgemäß ist in der Regel lediglich ein Teil der 21 Hydroxygruppen eines ß-Cyclodextrins verethert.

Die Herstellung von Sulfoalkyethercyclodextrinen ist dem Fachmann geläufig und beispielsweise in US 5,134,127 oder WO 2009/134347 A2 beschrieben.

Sulfoalkyethergruppen werden bei Cyclodextrinen im Stand der Technik zur Erhöhung der Hydrophilie beziehungsweise Wasserlöslichkeit eingesetzt. Die Erfindung hat erkannt, dass die Sulfoalkylethergruppen in besonderem Maße zur Erhöhung der Stabilität des Komplexes aus Anthocyanidinen und dementsprechend substituierten β-Cyclodextrin beitragen und so die Lagerstabilität und Formulierbarkeit der besonders oxidationsempfindlichen Anthocyanidine wesentlich verbessern. Der erfindungsgemäße Komplex kann als lagerstabile wässrige Lösung oder Feststoff formuliert werden, wie unten noch näher gezeigt werden wird.

Erfindungsgemäß besonders bevorzugt ist die Komplexierung mit einem Sulfobutylether-ß-Cyclodextrin (SEB-β-CD). Ein den Schutzbereich nicht beschränkender Erklärungsversuch hierfür ist, dass die negativ geladenen Sulfobutyleinheiten mit den positiv geladenen Anthocyanidinen elektrostatisch wechselwirken und unter den Alkylgruppen die Butylgruppe die optimale Länge besitzt, um sterisch eine entsprechende Wechselwirkung zu ermöglichen.

Bevorzugt beträgt der Substitutionsgrad des Cyclodextrins mit Sulfoalkylethergruppen 3 bis 8, weitervorzugsweise 4 bis 7. Geeignete Sulfobutylether-β-Cyclodextrine mit einem mittlerem Substitutionsgrad von 6 bis 7 sind beispielsweise in der genannten WO 2009/134347 A2 beschrieben und unter dem Handelsnamen Captisol® kommerziell erhältlich. Ebenfalls verwendbar sind entsprechende Cyclodextrine mit einem Substitutionsgrad von 4-5, beispielsweise 4,2.

Die erfindungsgemäß komplexierten Anthocyanidine sind bevorzugt ausgewählt aus der Gruppe bestehend aus Aurantinidin, Cyanidin, Delphinidin, Europinidin, Luteolinidin, Pelargonidin, Malvidin, Peonidin, Petunidin und Rosinidin. Die chemische Struktur entspricht der oben wiedergegebenen Formel I mit dem folgenden Substitutionsmuster

| | R^{3'} | R^{4'} | R^{5'} | R³ | R⁵ | R⁶ | R⁷ |
|---|---|---|---|---|---|---|---|
| Aurantinidin | -H | -OH | -H | -OH | -OH | -OH | -OH |
| Cyanidin | -OH | -OH | -H | -OH | -OH | -H | -OH |
| Delphinidin | -OH | -OH | -OH | -OH | -OH | -H | -OH |
| Europinidin | -OCH₃ | -OH | -OH | -OH | -OCH₃ | -H | -OH |
| Luteolinidin | -OH | -OH | -H | -OH | -OH | -H | -OH |
| Pelargonidin | -H | -OH | -H | -OH | -OH | -H | -OH |
| Malvidin | -OCH₃ | -OH | -OCH₃ | -OH | -OH | -H | -OH |
| Peonidin | -OCH₃ | -OH | -H | -OH | -OH | -H | -OH |
| Petunidin | -OH | -OH | -OCH₃ | -OH | -OH | -H | -OH |
| Rosinidin | -OCH₃ | -OH | -H | -OH | -OH | -H | -OCH₃ |

Besonders bevorzugt ist im Rahmen der Erfindung ein Komplex mit Delphinidin.

Gegenstand der Erfindung ist ferner eine wässrige Lösung eines erfindungsgemäßen Komplexes.

Ein weiterer Gegenstand ist ein Verfahren zur Herstellung eines solchen Komplexes sowie einer entsprechenden wässrigen Lösung, mit den Schritten:
a)Herstellen einer wässrigen Lösung des Sufoalkylether-β-Cyclodextrins,
b)Zugabe des Anthocyanidins und Vermischen zur Herstellung des Komplexes.

In Schritt a) wird bevorzugt eine wässrige Lösung hergestellt, die 5 bis 10 Gew.-% des verwendeten Cyclodextrins enthält. Besonders bevorzugt ist es im Rahmen der Erfindung, wenn der pH-Wert der wässrigen Lösung während oder nach, bevorzugt jedoch vor der Zugabe des Anthocyanidins, bevorzugt Delphinidins, auf einen pH-Wert von 7 oder weniger, vorzugsweise 6 oder weniger, weiter vorzugsweise 5 oder weniger, weiter vorzugsweise 4 bis 5, eingestellt wird. Es hat sich gezeigt, dass bei diesem pH-Wert sich eine höhere Konzentration des Komplexes in wässriger Lösung einstellen lässt.

Die Konzentration des Anthocyanidins berechnet als Chlorid, beträgt vorzugsweise wenigstens 0,5 mg/ml, weiter vorzugsweise wenigstens 1,0 mg/ml, weiter vorzugsweise wenigstens 1,5 mg/ml, weiter vorzugsweise 2,0 mg/ml beträgt. Der besonders bevorzugte Konzentrationsbereich von wenigstens 2,0 mg/ml lässt sich im Rahmen einer bevorzugten Ausführungsform insbesondere einstellen in einer wässrigen Lösung mit einem pH-Wert zwischen 4 und 5.

Im Rahmen der erfindungsgemäßen Herstellung kann das Vermischen der Bestandteile der wässrigen Lösung durch Rühren geschehen, bevorzugte Zeiträume für das vermischen sind 2 bis 20 h. Bevorzugt wird im Dunkeln gearbeitet, um eine lichtinduzierte Oxidation zu vermeiden.

Ein weiterer Gegenstand der Erfindung ist ein Feststoff enthaltend einen erfindungsgemäßen Komplex, der erfindungsgemäß erhältlich ist durch Entfernen des Lösungsmittels aus einer erfindungsgemäßen wässrigen Lösung. Das Entfernen kann bevorzugt durch Gefriertrocknung (Lyophilisation) erfolgen. Sowohl die erfindungsgemäße wässrige Lösung als auch der Feststoff besitzen eine hohe Lagerstabilität.

Ausführungsbeispiele der Erfindung werden nachfolgend erläutert.

### 1. Eingesetzte Materialien:

Die nachfolgenden Cyclodextrine werden verwendet:

| | |
|---|---|
| α-CD | ID No: CYL-2322 |
| β-CD | ID No: CYL-3190 |
| γ-CD | ID No: CYL-2323 |
| (2-Hydroxypropyl)-β-CD | ID No: L-043/07 |
| Sulfobutylether-β-CD | ID No: 47K010111 |

Delphinidinchlorid wurde von der Firma Extrasynthese erworben.

### 2. Bestimmung des Delphinidingehalts

Zur Bestimmung des Gehalts von Delphinidinchlorid in den delphinidinhaltigen Zusammensetzungen wurde ein Reverse phase HPLC-Verfahren verwendet. Dabei wurden folgende Reagenzien eingesetzt:
Gereinigtes Wasser
Methanol für die Chromatographie
Armeisensäure, p. a.
1 M Salzsäure als volumetrische Lösung.

Als Säule wurde eine Waters X Bridge™ C18,35 µl, 150 mm x 4,6 mm verwendet.

Die mobilen Phasen waren wie folgt:
Kanal A: Wasser 950 ml, Methanol 50 ml, Armeisensäure 10 ml
Kanal B: Wasser 50 ml, Methanol 950 ml, Armeisensäure 10 ml

Folgendes Gradientenprogramm wurde verwendet:

| Zeit [min] | Prozent Kanal B |
|---|---|
| 0 | 0 |
| 5 | 0 |
| 25 | 60 |
| 30 | 100 |

Stoppzeit: 35 min
   Nachlaufzeit (posttime): 8 min
Flussrate: 1 ml/min
Injektionsvolumen: 20 µl
Säulentemperatur: 30°C +/- 2°C
UV-Vis Detektor: 530 µm für den Assay, 275 µm zur Detektion von Verunreinigungen
Integrator: Fläche

### Lösungen und Probenvorbereitung:

- Verdünnungslösung 1:: Mischung aus 100 ml Methanol und 2,6 ml 1 M HCL
- Verdünnungslösung 2:: Mischung aus 100 ml 40 prozentiger Methanol und 2,6 ml 1 M HCL

Kalibrierungslösung: Eine Referenzlösung von Delphinidin wurde durch Einwiegen von 10 mg Delphinidinchlorid in einen 10 ml Kolben und Lösen in Verdünnungslösung 1 hergestellt. Nach dem Lösen wurde ungefähr 10fach verdünnt mit Verdünnungslösung 2 zur Herstellung einer ungefähren Konzentration von 0,1 mg/ml.

Die Kontrollkalibrierungslösung wurde auf die gleiche Art und Weise hergestellt. Die Kalibrierungslösungen wurden sofort mittels HPLC analysiert, da Delphinidinchlorid in Lösung instabil ist.

### Herstellung der Prüflösungen:

Zur Bestimmung des Delphinidingehalts erfindungsgemäß hergestellter Feststoffe (Herstellung siehe weiter unten) wurde etwa 50 mg dieser Zusammensetzung in einem 10 ml Kolben eingewogen. Anschließend wurde in Verdünnungslösung 2 gelöst und mit der gleichen Verdünnungslösung 2 weiter verdünnt bis zur Einstellung einer ungefähren Delphinidinkonzentration von 0,1 mg/ml.

Die Bestimmung des Delphinidingehalts in den Proben wurde berechnet unter Zuhilfenahme der Agilent ChemStation Software unter Verwendung der Kalibrierung mit dem beschriebenen externen Standard.

### Beispiel 1

### Komplexierung von Delphinidin mit SBE-β-CD.

In diesem Beispiel wird die Komplexierung von Delphinidin durch verschiedene Cyclodextrine und die Löslichkeit des Komplexes in wässriger Lösung untersucht. Die Komplexierung mit SBE-β-CD ist erfindungsgemäß, bei den übrigen Versuchen auf andere Cyclodextrine beziehungsweise Löslichkeit von Delphinidin (unkomplexiert) handelt sich um Vergleichsversuche.

Es wurden neutrale wässrige Lösungen hergestellt, die 10 Gew.-% des jeweiligen Cyclodextrins enthalten. Bei β-CD wurde auf Grund der mangelnden Löslichkeit eine Konzentration von lediglich 2 Gew.-% gewählt.

Je 5 ml der wässrigen Cyclodextrinlösungen und von reinem Wasser wurden in Glaskolben gefüllt. Anschließend wurde ein Überschuss Delphinidinchlorid zugegeben. Die erforderliche Überschussmenge war 10 mg für die Lösungen von α-, β- und γ-Cyclodextrin und 15 mg für die Lösungen von HPBCD (2-Hydroxypropyl-β-Cyclodextrin) und SBE-β-CD.

Die Suspensionen wurden für 20 h bei 30° C im Dunkeln gerührt. Anschließend wurde filtriert durch einen Membranfilter mit 0,22 µm Porengröße.

Die erzielbaren Löslichkeiten sind in der nachfolgenden Tabelle 1 wiedergegeben.

| Cyclodextrin | Cyclodextrin-Konzentration | Delphinidinchlorid |
|---|---|---|
| - | 0 | 0.07 mg/ml |
| α-CD | 10 % | 0.14 mg/ml |
| β-CD | 2 % | 0.05 mg/ml |
| γ-CD | 10 % | 0.21 mg/ml |
| HPBCD | 10 % | 0.19 mg/ml |
| SBE-ß-CD | 10 % | 0.66 mg/ml |

Man erkennt, dass die Komplexierung und dadurch bewirkte Löslichkeitserhöhung für SBE-β-CD weit besser ist als für die anderen Cyclodextrine.

### Beispiel 2 Einfluss des pH-Wertes

In diesem Beispiel wurde der Einfluss des pH-Wertes auf die Löslichkeit eines Delphinidin-SBE-β-CD in wässriger Lösung untersucht. Nach der Vorschrift von Beispiel 1 wurden wässrige Lösungen von SEB-β-CD hergestellt, diese Lösungen jedoch mit 1 M HCL auf die in Tabelle 2 genannten sauren pH-Werte eingestellt. Anschließend wurde Delphinidinchlorid nach der Vorschrift des Beispiels 1 zugegeben und weitergearbeitet, als einzige Abweichung wurde die Rührzeit auf 2,5 h begrenzt. Die Ergebnisse sind in der nachfolgenden Tabelle 2 wiedergegeben.

| pH | Delphinidinchlorid |
|---|---|
| 6.0 | 0.60 mg/ml |
| 4.8 | 2.12 mg/ml |
| 4.1 | 2.03 mg/ml |

Man erkennt, dass bei pH-Werten zwischen 4 und 5 die Löslichkeit des komplexierten Delphinidinchlorids sich etwa um den Faktor 3 gegenüber einem neutralen pH-Wert erhöht.

### Beispiel 3 Herstellung eines erfindungsgemäßen Feststoffs

In diesem Beispiel wird ein erfindungsgemäßer Komplex als Feststoff formuliert. Zu Vergleichszwecken werden ein Delphinidin/HPBCD Komplex sowie eine Delphinidin/Stärkeformulierung als Feststoff zubereitet.

### Beispiel 3.1: Delphinidin/SBE-β-CD

5 g SEB-β-CD wurden in 40 ml destilliertem Wasser zu einer klaren Lösung gelöst. Der pH-Wert der Lösung wurde mittels 1 M HCL auf 4,8 eingestellt. Anschließend wurden 0,11 g Delphinidinchlorid hinzugefügt und 2 h bei 27°C im Dunkeln gerührt. Die homogene Flüssigkeit wurde durch einen Cellulosenitratmembranfilter mit einer Porengröße von 0,45 µm vakuumfiltriert. Die Lösung wurde gefroren und anschließend gefriergetrocknet bei -48°C und einem Druck von etwa 10,3 Pa (77 mTorr). Das Lyophilisat wurde gemahlen und durch ein Sieb von 0,3 mm Maschenweite gesiebt.

### Beispiel 3.2: Delphinidin/HPBCD

Es wurde in gleicher Weise wie Beispiel 3.1 gearbeitet, jedoch wurde bei der Filtration eine signifikante Menge Material abfiltriert, was darauf hindeutet, dass die Solubilisierung deutlich weniger effektiv war als bei Verwendung von SBE-β-CD gemäß Beispiel 3.1.

### Beispiel 3.3 Delphinidin/Stärkeformulierung

5 g Stärke wurde in 40 ml destilliertem Wasser suspendiert. Man erhielt eine weiße Suspension. Der pH der Lösung wurde mit 1 M HCL auf 4,6 eingestellt. Anschließend wurde 0,11 g Delphinidinchlorid zugegeben und für 2 h bei 27° C im Dunkeln gerührt. Die erhaltene homogene Flüssigkeit wurde wie in Beispiel 3.1 gefriergetrocknet, gemahlen und gesiebt.

Beispiel 3.1 ist erfindungsgemäß, bei den Beispielen 3.2 und 3.3 handelt es sich um Vergleichsbeispiele.

### Beispiel 4 Stabilitätsversuche

Die Feststoffe gemäß den Beispielen 3.1 bis 3.3 wurden unter folgenden Bedingungen gelagert:
- 8 Tage bei Raumtemperatur in braunen, verschraubten Glasbehältern,
- Anschließend 22 Tage bei Raumtemperatur in Glasbehältern unter Sauerstoffatmosphäre im Dunkeln.

Die letzen 22 Tage der oben beschriebenen Lagerung wurden in Glasviolen mit einem Volumen 20 ml durchgeführt. Jeweils 250 mg der vorher bereits für 8 Tage gelagerten Proben wurden dort eingefüllt, die Violen wurden mit einem Gummistopfen verschlossen und abgedichtet. Mittels zwei Injektionsnadeln wurde der Kopfraum der Violen mit reinem Sauerstoff gespült. Die Proben wurden anschließend im Dunkeln gelagert.

Der Delphinidingehalt der Feststoffe (berechnet als Delphinidinchlorid und angegeben in Gew.-%) wurde mittels der oben beschriebenen HPLC-Methode bestimmt. Die Ergebnisse finden sich in der nachfolgenden Tabelle 3.

| | Zeitablauf [Tage] | | | | |
|---|---|---|---|---|---|
| | Start | 2 | 8 | 19 | 30 |
| Beispiel 3.1 | 1.69 | 1.52 | 1.55 | 1.40 | 0.93 |
| Beispiel 3.2 | 1.30 | 1.20 | 1.14 | 1.03 | 0.68 |
| Beispiel 3.3 | 1. 60 | 1.59 | 1.56 | 1.53 | 1.15 |

Die Ergebnisse zeigen, dass sich erfindungsgemäß ein Delpinidinkomplex herstellen lässt, der selbst unter reiner Sauerstoffatmosphäre eine hohe Stabilität und damit gute Lagerfähigkeit besitzt. Der Komplex besitzt ferner eine gute Löslichkeit in wässrigen, insbesondere leicht sauren Lösungen, so dass sich Delphinidin erfindungsgemäß auf vielfältige Art und Weise formulieren lässt. Die Stabilität des erfindungsgemäßen Feststoffes ist ähnlich gut wie eine Formulierung mit Stärke (Beispiel 3.3), dieses Vergleichsbeispiel lässt sich allerdings nicht in eine wässrige Lösung formulieren.

### Beispiel 5 Stabilitätsversuche in wässriger Lösung

Zur Bestimmung des Gehalts von Delphinidinchlorid in den delphinidinhaltigen Lösungen wurde ein Reverse phase HPLC-Verfahren ähnlich dem oben bereits beschriebenen verwendet. Dabei wurden folgende Reagenzien eingesetzt:
Gereinigtes Wasser
Methanol für die Chromatographie
Armeisensäure, p. a.
1 M Salzsäure als volumetrische Lösung.

Als Säule wurde eine Waters X Bridge™ C18,35 µl, 150 mm x 4,6 mm verwendet.

Die mobilen Phasen waren wie folgt:
Kanal A: Wasser 770 ml, Methanol 230 ml, Armeisensäure 10 ml
Kanal B: Wasser 50 ml, Methanol 950 ml, Armeisensäure 10 ml

Folgendes Gradientenprogramm wurde verwendet:

| Zeit [min] | Prozent Kanal B |
|---|---|
| 0 | 0 |
| 5 | 0 |
| 20 | 20 |
| 25 | 100 |

Stoppzeit: 25 min
Nachlaufzeit (posttime): 8 min
Flussrate: 1 ml/min
Injektionsvolumen: 20 µl
Säulentemperatur: 30°C +/- 2°C
UV-Vis Detektor: 530 µm für den Assay, 275 µm zur Detektion von Verunreinigungen
Integrator: Fläche

### Lösungen und Probenvorbereitung:

- Verdünnungslösung 1:: Mischung aus 100 ml Methanol und 2,6 ml 1 M HCL
- Verdünnungslösung 2:: Mischung aus 100 ml 50 %igem Methanol und 2,6 ml 1 M HCL

Kalibrierungslösung: Eine Referenzlösung von Delphinidin wurde durch Einwiegen von 10 mg Delphinidinchlorid in einen 10 ml Kolben und Lösen in Verdünnungslösung 1 hergestellt. Nach dem Lösen wurde ungefähr 10fach verdünnt mit Verdünnungslösung 2 zur Herstellung einer ungefähren Konzentration von 0,1 mg/ml.

Die Kontrollkalibrierungslösung wurde auf die gleiche Art und Weise hergestellt. Die Kalibrierungslösungen wurden sofort mittels HPLC analysiert, da Delphinidinchlorid in Lösung instabil ist.

### Herstellung der Prüflösungen:

Zur Bestimmung des Delphinidingehalts einer erfindungsgemäßen wässrigen Lösung wurden Delphinidin/SBE-β-CD des Beispiels 3.1 (erfindungsgemäß) und Delphinidin (Vergleichsbeispiel in 0,9 %iger NaCl-Lösung gelöst bis zur Einstellung einer Startkonzentration (bezogen auf das Delphinidin) von 1,584 mg/ml (erfindungsgemäßes Beispiel) bzw. 0,0216 mg/ml (Vergleichsbeispiel). Die Lösungen wurden bei Raumtemperatur hergestellt und anschließend bei 37°C im Dunkeln in verschlossenen Violen gelagert.

Nach 1, 2, 3 und 4 h wurde der Delphinidingehalt bestimmt. Die nachfolgende Tabelle gibt den ermittelten Gehalt als Prozentanteil der oben genannten Startkonzentration an.

| Zeit [h] | Delphinidin unkomplexiert | Delphinidin/SBE-β-CD |
|---|---|---|
| 0 | 100% | 100% |
| 1 | 8,3% | 80,7% |
| 2 | 6,5% | 74,5% |
| 3 | 5,6% | 64,7% |
| 4 | 5,1% | 62,8% |

Die Bestimmung des Delphinidingehalts in den Proben wurde berechnet unter Zuhilfenahme der Agilent ChemStation Software unter Verwendung der Kalibrierung mit dem beschriebenen externen Standard.

## Patentansprüche

1. Komplex aus einem Anthocyanidin und einem Sulfoalkylether-β-Cyclodextrin.

2. Komplex nach Anspruch 1, **dadurch gekennzeichnet, dass** das Sulfoalkylether-β-Cyclodextrin ein Sulfobutylether-β-Cyclodextrin (SBE-β-CD) ist.

3. Komplex nach Anspruch 1 oder 2, **dadurch gekennzeichnet, dass** der Substitutionsgrad des Cyclodextrins mit Sulfoalkylethergruppen 3 bis 8, vorzugsweise 4 bis 7 beträgt.

4. Komplex nach einem der Ansprüche 1 bis 3, **dadurch gekennzeichnet, dass** die Anthocyanidine ausgewählt sind aus der Gruppe bestehend aus Aurantinidin, Cyanidin, Delphinidin, Europinidin, Luteolinidin, Pelargonidin, Malvidin, Peonidin, Petunidin und Rosinidin.

5. Komplex nach Anspruch 4, **dadurch gekennzeichnet, dass** das Anthocyanidin Delphinidin ist.

6. Wässrige Lösung eines Komplexes nach einem der Ansprüche 1 bis 5.

7. Wässrige Lösung nach Anspruch 6, **dadurch gekennzeichnet, dass** sie einen pH-Wert von 7 oder weniger, vorzugsweise 6 oder weniger, weiter vorzugsweise 5 oder weniger, weiter vorzugsweise 4 bis 5, aufweist.

8. Wässrige Lösung nach Anspruch 6 oder 7, **dadurch gekennzeichnet, dass** die Konzentration des Anthcyanidins, berechnet als Chlorid, wenigstens 0,5 mg/ml, weiter vorzugsweise wenigstens 1,0 mg/ml, weiter vorzugsweise wenigstens 1,5 mg/ml, weiter vorzugsweise wenigstens 2,0 mg/ml beträgt.

9. Feststoff enthaltend einen Komplex aus einem Anthocyanidin und einem Sulfoalkylether-β-Cyclodextrin, erhältlich durch Entfernen des Lösungsmittels aus einer wässrigen Lösung nach gemäß einem der Ansprüche 6 bis 8.

10. Verfahren zur Herstellung eines Komplexes aus einem Anthocyanidin und einem Sulfoalkylether-β-Cyclodextrin, mit den Schritten:
a) Herstellen einer wässrigen Lösung des Sulfoalkylether-β-Cyclodextrins,
b) Zugabe des Anthocyanidins und Vermischen zur Herstellung des Komplexes.

11. Verfahren nach Anspruch 10, **dadurch gekennzeichnet, dass** die in Schritt a) hergestellte Lösung 5 bis 10 Gew.-% des Sulfoalkylether-β-Cyclodextrins enthält.

12. Verfahren nach Anspruch 10 oder 11, **dadurch gekennzeichnet, dass** der pH-Wert der in Schritt a) hergestellten Lösung vor der Zugabe des Anthocyanidins auf einen pH-Wert von 7 oder weniger, vorzugsweise 6 oder weniger, weiter vorzugsweise 5 oder weniger, weiter vorzugsweise 4 bis 5, eingestellt wird.

13. Verfahren nach einem der Ansprüche 10 bis 12, **dadurch gekennzeichnet, dass** das Vermischen in Schritt b) über einen Zeitraum von 2 bis 20 h stattfindet.

## Claims

1. Complex formed of an anthocyanidin and a sulfoalkyl ether β-cyclodextrin.

2. Complex according to claim 1, **characterised in that** the sulfoalkyl ether β-cyclodextrin is a sulfobutyl ether β-cyclodextrin (SBE-β-CD).

3. Complex according to either claim 1 or claim 2, **characterised in that** the degree of substitution of the cyclodextrin with sulfoalkyl ether groups is of 3 to 8, preferably of 4 to 7.

4. Complex according to any of claims 1 to 3, **characterised in that** the anthocyanidins are selected from the group consisting of aurantinidin, cyanidin, delphinidin, europinidin, luteolinidin, pelargonidin, malvidin, peonidin, petunidin and rosinidin.

5. Complex according to claim 4, **characterised in that** the anthocyanidin is delphinidin.

6. Aqueous solution of a complex according to any of claims 1 to 5.

7. Aqueous solution according to claim 6, **characterised in that** it has a pH of 7 or less, preferably of 6 or less, more preferably of 5 or less, more preferably of from 4 to 5.

8. Aqueous solution according to either claim 6 or claim 7, **characterised in that** the anthocyanidin is at a concentration, expressed as chloride, of at least 0.5 mg/ml, more preferably of at least 1.0 mg/ml, more preferably of at least 1.5 mg/ml, more preferably of at least 2.0 mg/ml.

9. Solid containing a complex formed of an anthocyanidin and a sulfoalkyl ether β-cyclodextrin, obtainable by removing the solvent from an aqueous solution according to any of claims 6 to 8.

10. Process for preparing a complex formed of an anthocyanidin and a sulfoalkyl ether β-cyclodextrin, comprising the steps of:
a) preparing an aqueous solution of the sulfoalkyl ether β-cyclodextrin,
b) adding the anthocyanidin and mixing in order to prepare the complex.

11. Process according to claim 10, **characterised in that** the solution prepared in step a) contains from 5 to 10 wt.% of the sulfoalkyl ether β-cyclodextrin.

12. Process according to either claim 10 or claim 11, **characterised in that** the pH of the solution prepared in step a) is adjusted before the addition of the anthocyanidin to a pH of 7 or less, preferably of 6 or less, more preferably of 5 or less, more preferably of from 4 to 5.

13. Process according to any of claims 10 to 12, **characterised in that** the mixing in step b) takes place over a period of from 2 to 20 hours.

## Revendications

1. Complexe constitué d'une anthocyanidine et d'une sulfoalkyléther-β-cyclodextrine.

2. Complexe selon la revendication 1, **caractérisé en ce que** la sulfoalkyléther-β-cyclodextrine est une sulfobutyléther-β-cyclodextrine (SBE-β-CD).

3. Complexe selon la revendication 1 ou 2, **caractérisé en ce que** le degré de substitution de la cyclodextrine comportant des groupes sulfoalkyléther est de 3 à 8, de préférence de 4 à 7.

4. Complexe selon l'une des revendications 1 à 3, **caractérisé en ce que** les anthocyanidines sont choisies dans le groupe consistant en l'aurantinidine, la cyanidine, la delphinidine, l'europinidine, la lutéolinidine, la pélargonidine, la malvidine, la péonidine, la pétunidine et la rosinidine.

5. Complexe selon la revendication 4, **caractérisé en ce que** l'anthocyanidine est la delphinidine.

6. Solution aqueuse d'un complexe selon l'une des revendications 1 à 5.

7. Solution aqueuse selon la revendication 6, **caractérisée en ce qu'**elle présente un pH de 7 ou moins, de préférence de 6 ou moins, plus préférentiellement de 5 ou moins, plus préférentiellement de 4 à 5.

8. Solution aqueuse selon la revendication 6 ou 7, **caractérisée en ce que** la concentration de l'anthocyanidine, calculée sous forme de chlorure, est d'au moins 0,5 mg/ml, plus préférentiellement d'au moins 1,0 mg/ml, plus préférentiellement d'au moins 1,5 mg/ml, plus préférentiellement d'au moins 2,0 mg/ml.

9. Matière solide contenant un complexe d'une anthocyanidine et d'une sulfoalkyléther-β-cyclodextrine, pouvant être obtenue par élimination du solvant à partir d'une solution aqueuse selon l'une des revendications 6 à 8.

10. Procédé de préparation d'un complexe d'une anthocyanidine et d'une sulfoalkyléther-β-cyclodextrine, comportant les étapes suivantes :
a) préparation d'une solution aqueuse de la sulfoalkyléther-β-cyclodextrine,
b) addition de l'anthocyanidine et mélange pour préparer le complexe.

11. Procédé selon la revendication 10, **caractérisé en ce que** la solution préparée dans l'étape a) contient 5 à 10 % en poids de la sulfoalkyléther-β-cyclodextrine.

12. Procédé selon la revendication 10 ou 11, **caractérisé en ce que** le pH de la solution préparée dans l'étape a) est, avant l'addition de l'anthocyanidine, ajusté à un pH de 7 ou moins, de préférence de 6 ou moins, plus préférentiellement de 5 ou moins, plus préférentiellement de 4 à 5.

13. Procédé selon l'une des revendications 10 à 12, **caractérisé en ce que** le mélange de l'étape b) est mis en oeuvre sur une durée de 2 à 20 h.
